# EUROPEAN PATENT APPLICATION

(11) **EP 2 450 075 A1**
(43) Date of publication of application: **09.05.2012**
(21) Application number: 11008614.7
(22) Date of filing: 27.10.2011
(51) Int. Cl.: A61M 21/00, A61N 5/06, A61N 1/36

(54) **Apparatus for delivering energy to a patient's body on the basis of the patient's biological parameters**

(30) Priority: 03.11.2010 IT MI20102038
(71) Applicant: Palmieri, Beniamino, I-41100 Modena (IT); Elettronica Valseriana S.R.L., 24020 Casnigo (BG) (IT)
(72) Inventor: Palmieri, Beniamino, 41100 Modena (MO) (IT)
(74) Representative: Gatti, Enrico

(57) **Abstract**

The apparatus (14) for treating a patient's body by delivering energy, comprises: a device (18) for delivering energy to the body of a patient (10); a series of sensors (S1-S6) for measuring all or part of the essential physiological integrated biorhythms of the patient (10); a control and command unit (20) enabling the energy delivered to the body of the patient (10) to be varied on the basis of the measurement by the sensors (S1-S6).

## Description

The present invention relates to an apparatus for treating a patient's body by energy delivery.

Current methods of delivering energy to a patient (in particular ultrasound, electrotherapy or magnetotherapy treatments, laser, radiofrequency, pulsating light, pressotherapy, iontophoresis or ionophoresis treatments) take no account of the conditions of the patient's essential biological parameters. The relative energy is administered to the patient independently of the patient's receptivity conditions at that moment, which in particular determine the rise in cell temperature and entropy, from which it is hoped, by previous experience, that the patient will derive beneficial effects.

All treatments involving the delivery of energy, in the various aforesaid forms, to a tissue, a limb or a body part (viscera) of a patient suffer from the limitation of not having any relationship with the patient's essential physiological integrated biorhythms, and hence an energy transfer which is neither functionally correlated nor commensurate with the physiopathological unbalance and the specific requirements of that patient for that determined illness at that moment.

The illnesses for which energy administration is used have as their common essential characteristic the loss of integrated synchronization of the circular, respiratory or neurological biorhythms, so compromising or limiting the relative function diminution in the cellular-subcellular energy required to quickly restore functional integrity. In the most serious cases this absence of reparative energy can result in cellular death and gradual functional stupor or meiopragy of variable duration. In this respect, a traumatized or edematous or contused limb, or one with a hematoma, which is treated with ultrasound or electrotherapy or other types of energy, was found to lose, in that segment the subject of the trauma, its own compliance with the biorhythms always present in physiological conditions in the body; transudate or exudate or hematic liquid can in fact be observed for example by extravasation in tissues as a result of secretion by proinflammatory cells (leucocytes, basophils, eosinophils, monocytes and macrophages) and also adrenergic monoamines which act via the central and peripheral nervous system and the adrenal glands. All these factors cause biorhythm interruption in the seat concerned, creating an inflammatory engorgement with vasoconstriction and/or vasodilation, itself causing stasis and aggravation of liquid and cell blockage.

From research and experiments carried out, it has been surprisingly found that in delivering the energy relative to a determined treatment, its administration synchronously with the essential physiological integrated biorhythms in order to potentiate them enables maximum effectiveness to be obtained together with maximum restoration of wellbeing and healing of the subject.

The object of the present invention is therefore to provide an apparatus which enables this latter result to be obtained.

Before explaining how this result is achieved by virtue of the present invention, it should be noted that the term "essential physiological integrated biorhythms" (EPIB) comprises:

### - CARDIAC AND ARTERIAL PULSATILITY

Measurable by pulse oximeters, phletismographs, oscillometers.

### - RESPIRATORY RHYTHM AND DEPTH

Measurable by thoracic impedancemetry and correlatable with oxygenation and CO₂ discharge.

### - CENTRAL ARTERIAL AND VENOUS PRESSURE

The first measurable by a pneumatic armband of the type conventionally used for holter pressure examinations and the second by strain gauge transducers.

### - GLOBAL CELL AND TISSUE ELECTRICAL ACTIVITY

Measurable by bioresonance sensors.

### - CUTANEOUS ELECTRICAL RESISTANCE AND CONDUCTANCE

Measurable respectively by ohmmeters and hygrometers (because also related to perspiration).

### - FUNCTIONAL RHYTHMS OF THE PERIPHERAL CENTRAL NERVOUS SYSTEM OF ORTHO AND PARASYMPATHETIC VOLUNTARY TYPE

Measurable by subjective perception of the working noise of the vital organs and in particular of the heart, caused by the flow of blood through the arteries; pulmonary rustling and abdominal rumblings. This object is attained by the apparatus of the present invention, comprising:
- a device for delivering energy to a patient's body;
- a series of sensors for measuring all or part of the patient's essential physiological integrated biorhythms;
- a control and command unit enabling the energy delivered to the patient's body to be varied on the basis of the sensor measurements. During the tests carried out, it was also surprisingly noted that if the patient is able to perceive with his/her senses the variation in the EPIB correlated parameters measured by the sensors, a particular concentration and knowledge of his/her state is generated in that patient, also influenceable by the wish of the subject, which causes variation in the patient's functional parameters and hence of the EPIBs (with a mechanism which we shall define as interactive biofeedback). Consequently the control and command unit acts to vary the energy delivered to the patient's body on the basis of the variation in said functional parameters.

Specific neuron paths have recently been identified in the cerebral amygdala structure which give rise to this concentration and knowledge phenomenon.

It should be noted that said interactive biofeedback has therapeutic mechanisms which are very different from the so-called placebo effect, i.e. from brain endorfin release (which reduce pain perception and increase the subjective wellbeing effect by virtue of the expectation of treatment benefits and the subjective conviction that it will be effective). In this respect the placebo effect is involuntary and unconscious, whereas the interactive feedback is directed to the intentional recruitment of all those forces and resources (endorphin secretion, vasodilation, direct hyperemia of an organ or at a limb, incretion of steroids and monoamines by the adrenal glands) required to implement the therapeutic effectiveness of energy delivered to the patient, for example laser energy, administered extrinsically. It is convenient that the said energy delivery (nearly always invisible, such as infrared laser energy) be concretely represented such that the patient perceives it by relating it to luminous and light effects (for example produced by means such as a screen or obtained by LEDs) which are able to achieve a reinforcement of the therapeutic effect by the induction of that known as chromotherapy; and/or acoustic effects (for example produced by amplifiers such as loudspeakers, headphones or earpieces); and/or effects perceivable through the skin (for example an air current the temperature of which varies as the delivered energy varies, or by cutaneus thermoceptors), and in any event effects perceivable by the patient's sensorial receptors which are able to reflectedly evoke biological responses synergic with the therapeutic action of the delivered energy.

To benefit from this situation, the apparatus of the invention is provided with means which enable the patient to perceive the variation in the parameters measured by the sensors, in particular visually (in particular via said screen) and/or by hearing (in particular via loudspeakers, headphones or earpieces), and possibly also by the other senses, and relative means for patient perception via these senses. It has been verified that this potentiates the therapeutic action of the energy delivered to the patient.

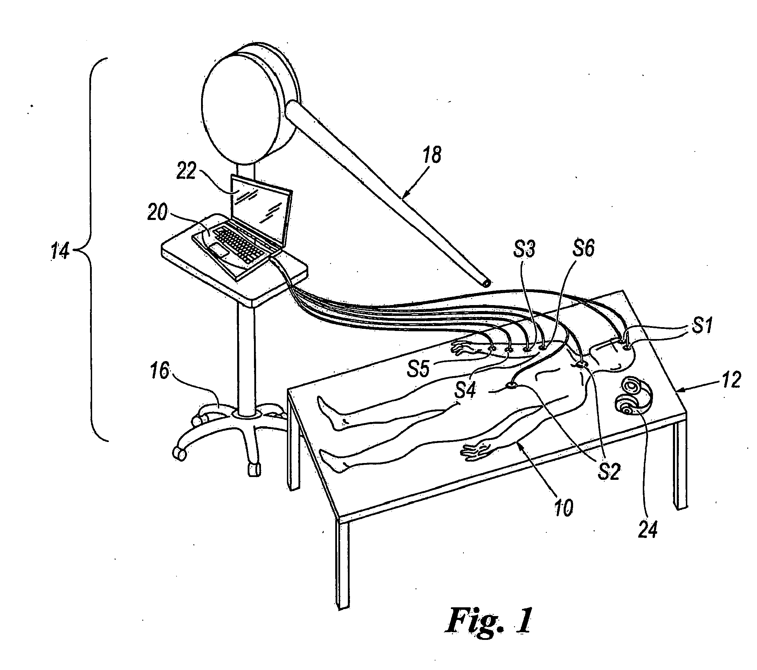

The invention will be more apparent from the ensuing description of one embodiment thereof, relating in particular to an apparatus for delivering energy to a patient in the form of powerless laser beams, hence not perceptible by the patient's senses. In this description reference will be made to the accompanying drawings, in which Figure 1 is a very schematic representation of a patient subjected to the action of said laser beam apparatus.

This figure shows a patient 10 lying on a bed 12 or other means for accommodating the patient 10 in the most comfortable manner. The same figure also shows very schematically an apparatus 14 for delivering light of powerless laser type comprising, in addition to a stand 16 carrying a multidiode device 18 (for example with helium neon guide light) able to emit energy in the form of infrared laser beams, this energy in the specific illustrated example being directed onto a particular region of the right arm of the patient 10.

The apparatus 14 also comprises a control and command unit 20 (in particular a microprocessor and preferably a personal computer) able to vary the energy delivered by the laser device 18 on the basis of the signals from a plurality of sensors able to measure all or part of the patient's essential physiological integrated biorhythms. In particular, in the specific illustrated example the sensors S1 are electroencephalographic transducers, the sensors S2 are ultrasound microamplifiers for perception of vital mechanical noises of the patient (in particular breathing, cardiac and visceral noises), S3 is a hygroscopicity sensor, S4 is an impedance meter, S5 is a pulsometry transducer and S6 is an arterial and/or venous pressure transducer. This latter is in particular of the type comprising a pneumatic armband connected to a transducer, commonly available for holter pressure examinations. The arterial pressure measurement becomes important in the case of patients with hypertension or are treated for cardiovascular problems or are stimulated at the charka points (essential psychophysical energy points according to oriental tradition). In this respect as certain types of hypertension can be modulated by psychophysical relaxation and voluntary breathing control, the final mechanism of which results in peripheral vasodilation, pressure regulation involves a blood flow increase in the peripheral districts and in the treated areas, with potentiated energy absorption.

The aforelisted sensors are connected by relative cables (although other known types of signal transmission means can be used) to the control and command unit 20 via a suitable decoder (no shown for simplicity). The unit 20 has been previously programmed to vary the emitted energy quantity, and in particular the quality (frequency) and intensity (i.e. the Joule value) of the energy irradiated as the parameters measured by the sensors S1-S6 vary. The variations are controlled such that the energy irradiated is a minimum when there is slow-down in the blood flow (expiratory and diastolic pauses) and progressively increases from the beginning of treatment as the integrated sensors gradually indicate patient relaxation (in particular cardiac slow-down, voluntary breathing slow-down, reduced electrical skin resistance, less surface hygroscopicity).

The irradiation frequency varies starting from a continuous initial emission stage, then increasing the frequency during the "energy loading" stage of the figured circulating blood elements, such as diastole and inspiration, but also the other parametral variations which enable quicker energy loading.

The variation in energy emitted by the laser device 18 of multidiode type (i.e. having a plurality of energy emission sources, in practice up to 20 diodes) can be achieved by selecting one or more diodes by a q-switched device.

The apparatus 14 also comprises a screen 22 on which the patient sees the variation in said parameters represented, for example by polychrome representations (chromotherapeutic).

As already stated, it is precisely the fact that the patient can observe and follow the variation in said parameters which gives rise to the aforesaid interactive biofeedback, which is extremely important.

The variation in delivered energy can also be shown on the same screen such that this variation can be perceived by the patient, this also substantially contributing to said interactive feedback. This energy variation can also be perceived by the patient in other ways, for example by an air current the variable intensity of which stimulates the patient's cutaneous pressure receptors, or by multipoint means which induce a variable pricking sensation, or by small electrical discharges of variable intensity, or an air flow (preferably ionized) at variable temperature, such that the skin, as the receptive sensorial member, sends stimuli to the brain which evoke reflexive neuronal responses aimed at soothing, attenuating, reducing inflammation or deflating the injured part.

Figure 1 shows, resting on the bed 12, headphones 24 which can be used by the patient to prevent excessive environmental noise contamination, to hence perceive the physiological body noises directly in the headphones.

As an example of the use of the apparatus 14 to deliver energy in the form of laser light, a patient 10 suffering from scapulo-humeral periarthritis is treated. Having activated the apparatus 14 and in particular the multidiode device 18, we shall assume that the sensors (in particular the sensors S2 and S5) measure a frequency of 78 heartbeats/minute and respiration of 18 breaths/minute, values which are fed to the control and command unit 20. We shall also assume that the control and command unit is programmed for example to cause the laser device 18 to radiate an energy of 15 Joule/m² divided into 20 cycles/second as frequency during the diastole and into 4 cycles/second during the systole, with an initially monodiode intensity which progressively increases to arrive for example at an energy value up to 20 times the initial value as the electrical conductivity of the skin measured by the sensor S4 gradually rises from 3 to 10 kOhm and the brain alpha waves increase from 2 to 8 cycles/second, while the surface hygroscopicity measured by the sensor S3 falls from 6 to 2 (using the commonly used reference values based on the evaluation of the dielectric constant of the skin corneal layer), final values which clearly indicate a state of relaxation of the patient.

This increase in radiated laser energy is obtained by gradually activating an increasing number of diodes up to the maximum number available, as a result of the fact that the patient 10 progressively attains the state of maximum relaxation by listening, for example through the headphones 24 (or through other amplifiers with which the apparatus 14 is provided) to the mechanical noises of the patient's own vital activity while at the same time viewing on the screen 22 (preferably in a chromatic scale based on chromatography principles) the patient's own parameters and also, on the same screen, the representation in similar terms of the quantity of laser energy which is gradually radiated onto his/her shoulder. The patient's attention is inevitably concentrated on the relative part of his/her body, which perceives a growing heat sensation due to the radiated laser energy which the (suitably programmed) unit 20 causes to increase as the patient's relaxation increases.

The apparatus 14 can also be provided with a source of ionized air which during radiation strikes the patient's shoulder, the temperature of which being made to increase by the unit 20 as relaxation increases.

The programming of the unit 20 can comprise a determined treatment duration once the patient has reached relaxation, with a maximum duration which must not be exceeded.

During the last treatment stage (for example the last three minutes), the delivered laser energy is suitably reduced gradually (such that at the end there is for example only one diode active), so consequently returning the patient into a deconcentration stage and activating active and/or passive articular movements to evaluate the symptomatology improvement by comparing the pre-post treatment situations, for example by the pain scale (such as the Scott-Husskinson visual analogue).

As already stated, the energy delivered by the apparatus of the present invention can be of mechanical type and in particular can be an apparatus for pressure therapy, which in addition to the aforesaid sensors and to the control and command unit is provided with pneumatic sleeves for the limbs, which can be inflated by a pressure pump. Said sleeves are specifically two leg sleeves, with the apparatus serving to facilitate the return circulation of the lower limbs. The control and command unit is programmed such that inflation occurs in two steps in accordance with the systole: a pre-pulse up to 80-100 mmHg during the diastole and a strong pulse up to 20 mmHg beyond maximum pressure during the systole, with a particular prolonged reinforcement shot lasting 10-20 seconds at high pressure (even 200 mmHg) during the expiratory pause and minisurplus trains of pulsating pressure (with 2.3 cycles/second frequency) during inspiration.

This apparatus mainly uses the heart-breathing-pressure sensors (S5, S2, S6 respectively), however the other sensors serve to cause the inflation pressure to increase progressively as the patient's relaxation increases, seeing that relaxation causes vasodilation which requires greater pressure, as a larger dilated and hence inert vascular bed must be achieved.

This articulated passive pneumatic exercise with the integrated biosensors S1-S6 serves to at least partly restore an arteriovenous lymphatic pulsating flow where this has been lost, causing leg swelling.

Finally it should be noted that the device for delivering energy to the patient can be not only of the laser energy or mechanical energy delivery type but also of a type able to deliver other forms of energy, for example in the form of ultrasound or magnetic field or radiofrequency, or by iontophoresis or ionophoresis or pulsating light.

## Claims

1. An apparatus (14) for treating the body of a patient (10) by delivering energy, comprising:
- a device (18) for delivering energy to the body of a patient (10);
- a series of sensors (S1-S6) for measuring all or part of the essential physiological integrated biorhythms of the patient (10);
- a control and command unit (20) enabling the energy delivered to the body of the patient (10) to be varied on the basis of the measurement by the sensors (S1-S6).

2. An apparatus (14) as claimed in claim 1, wherein the unit (20) provides for increasing the delivered energy as a result of the measurement by the sensors (S1-S6) of values which indicate the attainment of a relaxation state by the patient (10).

3. An apparatus (14) as claimed in claim 1, wherein means (22, 24) are provided which enable the patient (10) to perceive the variations in the parameters measured by the sensors (S1-S6).

4. An apparatus (14) as claimed in claim 3, wherein means (22) are provided which enable the patient (10) to perceive the variations in the energy delivered by the delivery device (18).

5. An apparatus (14) as claimed in claim 3, wherein means which enable the patient (10) to perceive the variations in the parameters measured by the sensors (S1-S6) comprise visual means (22) and/or acoustic means (24) and/or means which act on the cutaneous perception of the patient (10).

6. An apparatus (14) as claimed in claim 1, wherein the sensors are electroencephalographic transducers (S1), ultrasound microamplifiers (S2) for perception of the vital mechanical noises of the patient (10), hygroscopicity sensors (S3) for the skin of the patient (10), impedance meters (S5) applied to the skin of the patient (10), pulsometry transducers (S5) and arterial and/or venous pressure transducers (S6).

7. An apparatus (14) as claimed in claim 1, wherein the energy delivery device (18) is of the type for delivering infrared laser light.

8. An apparatus (14) as claimed in claim 7, wherein the infrared laser light device (18) is multidiode.

9. An apparatus (14) as claimed in claim 1, wherein the control and command unit is a personal computer (20).

10. An apparatus (14) as claimed in claim 5, wherein the visual means comprise a screen (22) on which the parameters measured by the sensors (S1-S6) are polychrome representations.

11. An apparatus (14) as claimed in claim 10, wherein the variation in the energy delivered to the patient (10) is also represented on the screen (22).

12. An apparatus (14) as claimed in claim 5, wherein cutaneous perception means are also provided, enabling the patient (10) to perceive the variation in delivered energy.

13. An apparatus (14) as claimed in claim 12, wherein the cutaneous perception means comprise an air current which strikes that part of the body of the patient (10) under treatment, the temperature of which increases as the energy delivered increases.

14. An apparatus (14) as claimed in any one of claims 1 to 6. wherein the device for delivering energy to the patient (10) is of the type for delivering mechanical energy.

15. An apparatus (14) as claimed in claim 14. wherein the mechanical energy delivery device is a device for pressotherapy provided with inflatable sleeves for the limbs, and wherein the control and command unit (20) is programmed such that the inflation is in accordance with the systole.

16. An apparatus (14) as claimed in any one of claims 1 to 6. wherein the device for delivering energy to the patient (10) is of the type for delivering energy in the form of ultrasound or of electric current or of magnetic field or of radiofrequency or of iontophoresis or ionophoresis.
